(19) Europäisches Patentamt
European Patent Office
Office européen des brevets

(11) EP 0 872 232 A1

(12) EUROPÄISCHE PATENTANMELDUNG

(43) Veröffentlichungstag:
21.10.1998  Patentblatt 1998/43

(51) Int. Cl.⁶: **A61K 9/06**, A61K 31/71, A61K 31/65, A61K 31/505, A61K 47/00, A61K 45/08

(21) Anmeldenummer: 98106425.6

(22) Anmeldetag: 08.04.1998

(84) Benannte Vertragsstaaten:
AT BE CH CY DE DK ES FI FR GB GR IE IT LI LU MC NL PT SE
Benannte Erstreckungsstaaten:
AL LT LV MK RO SI

(30) Priorität: 10.04.1997 ES 9700765

(71) Anmelder: BASF Labiana S.A.
08228 Les Fonts - Tarrasa (ES)

(72) Erfinder:
• Tico Grau, Jose Ramon
08034 Barcelona (ES)
• Suné Negre, Jose Maria
08028 Barcelona (ES)

(54) **Pharmazeutische Zubereitung mit verzögerter Wirkstofffreigabe**

(57) Die Erfindung betrifft eine pharmazeutische Zubereitung mit verzögerter Wirkstofffreigabe und deren Verwendung zur Herstellung von Injektionslösungen zur therapeutischen Behandlung von Tieren.

**Beschreibung**

Die Erfindung betrifft eine pharmazeutische Zubereitung mit verzögerter Wirkstofffreigabe und deren Verwendung zur Herstellung von Injektionslösungen zur therapeutischen Behandlung von Tieren.

In GB-A-2 136 293 wird beispielsweise ein entzündungshemmendes Mittel aus Tylosin und Steroiden als Wirkstoffe und einem Desinfektionsmittel beschrieben. DE-A-32 33 661 ist eine Injektionslösung zu entnehmen, die neben einem ölgelösten Wirkstoff Maleinsäure oder ein Salz der Maleinsäure enthält.

Von Armstrong et al (US 4,018,889) wird eine wäßrige Lösung von Oxytetracyclin mit 2-Pyrrolidon als Cosolvenz beschrieben. Nachteil der genannten Zubereitungen ist, daß sie nur über einen relativ kurzen Zeitraum den Wirkstoff freisetzen, so daß häufig neue Injektionen bzw. Applikationen erforderlich sind. Diese häufigen Injektionen bzw. Applikationen führen sehr leicht zu Läsionen der Haut oder Schleimhaut. Darüber hinaus entmischen sich die Zubereitungen aufgrund der geringen Löslichkeit der Wirkstoffe sehr rasch. Sie müssen deshalb von der Verabreichung intensiv gemischt werden. Bei längerer Lagerung verkleben die Wirkstoffe, so daß trotz intensiver Mischung größere Wirkstoffpartikel erhalten bleiben, die die Injektionsnadel verstopfen können.

Von Patterson et al wird in WO 96/01634 ein Oxytetracyclinpräparat mit verzögerter Wirkstoffabgabe beschrieben. Zur Herstellung dieses Präparates wird Glycerinformal verwendet. Glycerinformal besitzt toxikopharmakologische Eigenschaften und ist deshalb für pharmazeutische Anwendungen ungeeignet. So ist es beispielsweise teratogen und wirkt wachstumsverzögernd.

In WO 94/21267 wird eine pharmazeutische Zubereitung beschrieben, enthaltend den Wirkstoff Tylosin in einer öligen Matrix sowie ein gelbildendes Mittel, aus der der Wirkstoff über einen längeren Zeitraum freigesetzt wird.

Auch diese Zubereitung entmischt sich sehr rasch und zeigt ein Verkleben des Wirkstoffes nach dem Sedimentieren. Dies führt dazu, daß die Injektionslösung nur schwer redispergierbar ist und bei Injektion sich die Injektionsnadel zusetzen kann. Bei längerer Lagerung dieser Lösung verstärken sich diese genannten Probleme deutlich, so daß die Lösung nur eine geringe Lagerstabilität aufweist.

Eine optimale pharmazeutische Zubereitung sollte vorteilhafterweise zur Anwendung als Injektionslösung eine möglichst geringe Viskosität haben, so daß dünne Nadeln zur Applikation verwendet werden können, eine geringe Neigung zur Sedimentation zeigen, eine homogene Verteilung der Inhaltsstoffe aufweisen, sich nach längerer Lagerung leicht redispergieren lassen und einen möglichst hohen Wirkstoffgehalt haben, so daß nur geringe Volumina appliziert werden müssen.

Aufgabe der vorliegenden Erfindung war es, eine pharmazeutische Zubereitung mit verzögerter Wirkstofffreigabe zu entwickeln, die die in den oben aufgeführten Schriften genannten Nachteile nicht aufweist und die vorteilhafterweise trotz längerer Lagerung eine homogene Verteilung der Inhaltsstoffe und keine oder nur eine geringe Sedimentation aufweist. Darüber hinaus sollte sie sehr leicht redispergierbar sein und sich anschließend leicht injizieren lassen.

Diese Aufgabe wurde durch eine pharmazeutische Zubereitung mit verzögerter Wirkstofffreigabe gelöst, enthaltend:

a) einen Wirkstoff
b) ein gelbildendes Mittel
c) einen Puffer
d) eine grenzflächenaktive Substanz

Als Wirkstoff für die erfindungsgemäße Zubereitung sind prinzipiell alle in der Tierernährung und Tiermedizin verwendeten Wirkstoffe geeignet. Bevorzugt sind Wirkstoffe, die bei Infektionen durch Mikroorganismen eingesetzt werden, beispielsweise Tylosin, Tetracyclin, Oxytetracyclin, Gentamycin, Chloramphenicol, 2,4-Diaminobenzylpyrimidine, wie Trimethoprim, deren Salze oder deren Mischungen.

Besonders bevorzugt ist Tylosin (15-[[(6-desoxy-2,3-di-O-methyl-β-D-allopyranosyl)-oxy]-methyl]-6-[[3,6-didesoxy-4-O-(2,6-didesoxy-3-C-methyl-α-L-ribohexapyranosyl)-3-(dimethylamino)-β-D-glucopyranosyl]-oxy-16-ethyl-4-hydroxy-5,0-dimethyl-2,10-dioxo-oxacyclohexyl-deca-11,13-dien-7-acetaldehyd]) und seine Salze.

Unter Mischungen sind beispielsweise die Kombinationen von mehreren Wirkstoffen zur Verbesserung der allgemeinen Konstitution der Tiere sowie der Bekämpfung von Infektionen über z.B. Gabe von Vitaminen, Carotinoiden, Aminosäuren und/oder mindestens eines Antibiotikums zu verstehen. Vorteilhafterweise sind unter Mischungen von Wirkstoffen Mischungen, die mehrere Antibiotika mit unterschiedlicher Wirkung z.B. Antibiotika mit bakteriostatischer und/oder bakteriozider Wirkung wie die Kombination von Sulfonamiden mit Tylosin enthalten, und/oder die am gleichen Wirkort und/oder an unterschiedlichen Wirkorten wirken können, und/oder die aus unterschiedlichen Substanzklassen wie die Kombination von Tylosin mit Chloramphenicol bestehen, zu verstehen.

Bei Wirkstoffen, die sich in der erfindungsgemäßen Zubereitung wie z.B. Tylosin nicht lösen, sondern in Suspension vorliegen sollte die Partikelgröße möglichst gering sein um ein Verstopfen der Injektionsnadel bei parenteraler Applikation zu verhindern.

Die Partikelgröße der suspendierten Wirkstoffe sollte kleiner als 150 μm sein. Vorzugsweise sollte die Partikelgröße in einem Bereich zwischen 1 bis 100 μm liegen, besonders bevorzugt zwischen 1 und 50 μm, ganz besonders bevorzugt zwischen 1 bis 20 μm, wobei vorteilhafterweise großer 80 % der Teilchen in diesem Größenbereich liegen sollte.

Die Wirkstoffkonzentration in der Zubereitung liegt gewöhnlich im Bereich von 1 bis 40 Gew.-%, bevorzugt zwischen 5 bis 30 Gew.-% bezogen auf die gesamte Zubereitung.

Für die erfindungsgemäße Zubereitung sind prinzipiell alle gelbildenden Mittel geeignet. Vorteilhafterweise werden dabei gelbildende Mittel verwendet, die speziell für parenterale Applikationen geeignet sind. Dabei sollte das gelbildende Mittel die Viskosität der Zubereitung so erhöhen, daß die Inhaltsstoffe ausreichen gut d.h. homogen und langandauernd in der Zubereitung verteilt werden, gleichzeitig darf jedoch die Viskosität nicht so hoch werden, daß eine Injektion behindert wird. Vorteilhafterweise werden gelbildende Mittel verwendet, die thixotropische Eigenschaften aufweisen.

Vorteilhaft ist eine Viskosität zwischen 50 und 1800 cps (= centipoise, 1 cps = 1 mPa x s ) bevorzugt zwischen 50 und 1500 cps.

Die erfindungsgemäßen Zubereitungen sind in einer bevorzugten Ausführungsform thixotropisch, daß heißt ihre Viskosität verändert sich unter Einwirkung mechanischer Kräfte. Unter diesen Bedingungen verringert sich die Viskosität auf 50 bis 250 cps.

Als gelbildende Mittel kommen beispielsweise natürliche, modifizierte natürliche, synthetische und/oder anorganische Verdicker wie Agar-Agar, Tragant, Gummiarabicum, galacto-mannanhaltige Polysaccharide, Stärke, Cellulose, Carboxymethylcellulose, Poly(meth)acrylate, Polyvinylpyrrolidone, Polyether, Polykieselsäuren in Frage. Ebenfalls geeignet sind Gelatine, Pektine oder Metallseifen wie beispielsweise die Aluminium-, Calcium-, Lithium-, Zink- oder Magnesiumsalze von Fettsäuren, wie Aluminium-, Calcium-, Lithium- oder Magnesiumstearate.

Besonders bevorzugt sind die Aluminiumsalze von Fettsäuren wie z.B. die Aluminiummono-, -di- und/oder -tristearate.

Auch Mischungen verschiedener gelbildender Mittel eignen sich für die erfindungsgemäße Zubereitung.

Prinzipiell sind alle Puffer für die erfindungsgemäße Zubereitung geeignet, die den pH-Wert in einer ausreichenden Mengen in einem Bereich von pH 4 bis 10 halten, vorzugsweise zwischen 5 bis 9,5 besonders bevorzugt zwischen 6,8 bis 9,0.

Als Puffer seien hier beispielhaft Puffer organischer Säuren wie Succinat-, Acetat-, Maleat- oder Citratpuffer, Aminosäurepuffer, Phosphat-, Barbital- oder Trometamolpuffer (=Tris(hydroxymethyl)aminomethan, siehe DAB, Deutsche Arzneimittelbuch 1996, VII. 1.3 Pufferlösungen) genannt. Ebenfalls zum Puffern geeignet sind Puffer niederer primärer, sekundärer oder tertiärer Amine wie 2-Aminoethanol, Diethylamin, Ethylamin, Dimethylamin oder Triethanolamin.

Besonders geeignet sind die Trometamolpuffer. Die Puffer können als Lösung oder in Substanz verwendet werden. Auch Mischungen der Puffer können verwendet werden. Zur Verhinderung von Schmerzen bei der Injektion können der Zubereitung Anästhetika zugesetzt werden.

Als grenzflächenaktive Substanzen für die erfindungsgemäße Zubereitung eignen sich nichtionische, amphotere, anionische oder kationische Emulgatoren oder deren Mischungen, wie die Alkylarylpolyetheralkohole, oder Alkylphenylether des Polyethylenglykols (= Triton[®]-Marken) wie Triton[®]X-15 (1 Ethylenoxideinheit = EO), Triton[®]X-35 (3EO), Triton[®]X-45 (5EO), Triton[®]X-100 (9-10EO), die Sorbitanfettsäureester (Tween[®]- oder Span[®]-Marken) wie Tween[®]-20 (Polyoxyethylen(20)-sorbitanmonolaurat), Tween[®]-40 (Polyoxyethylen(20)-sorbitanmonopalmitat), Tween[®]-60(Polyoxyethylen(20)-sorbitanmonostearat), Tween[®]-80(Polyoxyethylen(20)-Sorbitanmonooleat), Span[®]-20(Sorbitanmonododecanat), Span[®]-40(Sorbitanmonohexadecanoat), Span[®]-60(Sorbitanmonooctadecanoat), Span[®]-80(Sorbitanmonooleat), Span[®]-85(Sorbitantrioleat), die Polyoxyethylenfettalkoholether (Brij[®]-Marken) wie Brij[®]56(Polyoxyethylen(10)cetylether), Brij[®]76(Polyoxyethylen(10)-stearylether), Brij[®]92(Polyoxyethylen(2)-oleylether) oder die Polyoxyethylenfettsäureester (Myrj[®]-Marken) wie (Myrj[®]-45(Polyoxyethylen(8)stearat), (Myrj[®]-52(Polyoxyethylen(40)stearat), Natriumlaurylsulfat.

Bevorzugt eignen sich die Sorbitanfettsäureester, besonders bevorzugt die Span[®]-Marken. Die grenzflächenaktiven Substanzen werden in einem Bereich von 0,05 bis 5 Gew.-% bevorzugt von 0,1 bis 2 Gew.-% verwendet.

Die erfindungsgemäße Zubereitung kann außerdem ein Lösungsmittel oder Lösungsmittelsystem enthalten.

Vorteilhafte Lösungsmittel oder Lösungsmittelsysteme sind ein- oder mehrwertige Alkohole, deren Ether, Ester oder deren Mischungen.

Beispielhaft seien Wasser, 2-Pyrrolidon, Benzylalkohol, Polyethylenglycol, Propylenglycol, Propylenglycoldicaprylat und/oder Propylenglycoldicaprat genannt. Bevorzugt sind Wasser, Benzylalkohol, Propylenglycoldicaprylat und/oder -dicaprat. Die Lösungsmittel bzw. Lösungsmittelsysteme werden in einem Bereich von 10 bis 95 Gew.-% bezogen auf die gesamte Zubereitung verwendet.

Vorteilhafterweise enthalten die erfindungsgemäßen Zubereitungen ein Lösungsmittel oder Losungsmittelsystem.

Darüber hinaus kann die erfindungsgemäße Zubereitung weitere Hilfsstoffe wie z.B. Antioxidantien wie Natriumbisulfit- oder Natriummetabisulfit, Konservierungsmittel, Netzmittel oder Stabilisatoren enthalten.

In einer bevorzugten Ausführungsform enthält die Zubereitung folgende Stoffe:

5,0 - 30 Gew.-% Tylosin
0,2 - 2 Gew.-% Benzylalkohol
0,1 - 3 Gew.-% Aluminium Tristearat
0,05 - 5 Gew.-% Span 85
0,1 - 3 Gew.-% Tris-Puffer
57 - 95 Gew.-% Propylenglycoldicaprylat/-dicaprat,

Die Summe der einzelnen Komponenten dieser bevorzugten Ausführungsform addiert sich zu 100 Gew.-%.

Die erfindungsgemäße Zubereitung kann direkt oder nach Zugabe von Wasser für alle parenteralen Applikationen bevorzugt als Injektionslösung für intramuskuläre, intravenöse oder subkutane Applikationen verwendet werden.

Die folgenden Beispiele dienen der weiteren Veranschaulichung der Erfindung.

Beispiele:

Die verschiedenen getesteten Zubereitungen sind den Tabellen I bis V zu entnehmen (Beispiele 1 bis 34). Die angegebenen Mengen sind in den Tabellen, wenn nicht anders erwähnt, in Gramm angegeben. Lösungsmittel oder Lösungsmittelsysteme wurden den Zubereitungen in einer Menge von 100 ml zugegeben.

Die verschiedenen Komponenten, der in den Beispielen beschriebenen Zubereitungen, wurden vorteilhafterweise nach folgendem Schema zusanmengefügt. Zunächst wurden der Wirkstoff Tylosin und das Trometamol alleine oder zusammen micronisiert (< 25 μm Partikelgröße) und anschließend sterilisiert. Das Lösungsmittel oder Lösungsmittelsystem beispielsweise Propylenglycoldicaprylat/-caprat wurde vorteilhaft auf 100 bis 150 °C erhitzt und das Aluminiumstearat langsam unter Rühren zugefügt. Danach wurde die oberflächenaktive Substanz beispielsweise Span-85 unter Rühren langsam zugefügt. Zu dieser Mischung wurde der Benzylalkohol gegeben. Prinzipiell können diese Substanzen auch in anderer Reihenfolge oder zusammen zugegeben werden. Nach Abkühlen der Mischung auf Temperaturen, die den Wirkstoff nicht mehr schädigen können, vorzugsweise 25 °C wurden der Wirkstoff Tylosin und das Trometamol einzeln oder zusammen zugegeben. Das Trometamol kann auch vor dem Abkühlen der Mischung zugesetzt werden. Anschließend wurde die Zubereitung vorteilhafterweise noch filtriert (100 μm Filter).

Die Injizierbarkeit der Zubereitungen wurde mit einer Spritze (12/10, 15/10 oder 18/10) bestimmt. Dazu wurden 5 ml oder 10 ml der angesetzten Zubereitungen nach den angegebenen Zeiten (siehe Tabellen VI bis XIII) in einer Spritze aufgenommen und ohne Unterbrechung durch eine Injektionsnadel unterschiedlicher Stärke 12/10, 15/10 oder 18/10 entleert und die Schwer- bzw. Leichtgängikeit oder ein Verstopfen der Nadel beurteilt.

Die Viskosität der Zubereitungen wurde nach einer Methode von Brookfield mit einem Viskosimeter der Firma Brookfield Engineering Laboratories, Inc. Stoughton, Mass, U.S.A. bestimmt. Dazu wurde ein Meßbecher/-glas mit der zumessenden Lösung gänzlich gefüllt und in einem Wasserbad auf 20 °C erwärmt. In die Losung wurde anschließend ein sich mit einer definierten Kraft und 30 Upm drehender Zylinder eingetaucht. Die Verzögerung der Drehgeschwindigkeit ist ein Maß für die Viskosität der Zubereitung und läßt sich in Centipoise ( 1 cps = 1 mPa x s ) umrechnen.

Die Höhe des sich gebildeten Sediments in den Zubereitungen wurde gemessen und bewertet (gering, keine oder gleichmäßig). Ebenfalls wurde die Bildungsgeschwindigkeit der Sedimente (schnell oder langsam) beurteilt.

Zur Beurteilung der Redispergierbarkeit der Zubereitungen wurden diese auf einen Schüttler (60 Upm) für 15 Minuten geschüttelt und anschließend die Redispergierbarkeit bewertet.

Tabelle I

| Unterschiedliche Partikelgröße des Wirkstoffes | | | |
|---|---|---|---|
| Zusammensetzung (Partikelgröße) | Beispiele | | |
| | 1 | 2 | 3 |
| Tylosin Base (50 to 100 μm >80%) | 25 g | - | - |
| Tylosin Base (25 to 50 μm >80%) | - | 25 g | - |
| Tylosin Base (5 to 20 μm >80%) | - | - | 25 g |
| Aluminiumtristearat | 0.500 g | 0.500 g | 0.500 g |
| Propylenglycol | 100 ml | 100 ml | 100 ml |

Tabelle II

| Grenzflächenaktive Substanzen | | | | | | | | | |
|---|---|---|---|---|---|---|---|---|---|
| Zusammensetzung | Beispiele | | | | | | | | |
| | 4 | 5 | 6 | 7 | 8 | 9 | 10 | 11 | 12 |
| Tylosin Base (5 to 20 $\mu$m) | 25 | 25 | 25 | 25 | 25 | 25 | 25 | 25 | 25 |
| Benzylalkohol | 0.9 | 0.9 | 0.9 | 0.9 | 0.9 | 0.9 | 0.9 | 0.9 | 0.9 |
| Aluminiumtristearat | 0.5 | 0.5 | 0.5 | 0.5 | 0.5 | 0.5 | 0.5 | 0.5 | 0.5 |
| Span 20 | - | - | - | - | - | - | 0.1 | 0.5 | 0.3 |
| Span 80 | - | - | - | 0.1 | 0.5 | 0.3 | - | - | - |
| Span 85 | 0.1 | 0.5 | 0.3 | - | - | - | - | - | - |
| Propylenglycoldicaprilat | 100ml | 100ml | 100ml | 100ml | 100ml | 100ml | 100ml | 100ml | 100ml |

Tabelle III

| pH-Kontrolle verschiedener Zubereitungen | | | | | | | | | |
|---|---|---|---|---|---|---|---|---|---|
| Zusammensetzung | Beispiele | | | | | | | | |
| | 13 | 14 | 15 | 16 | 17 | 18 | 19 | 20 | 21 |
| Tylosin Base (5 to 20 $\mu$m) | 25 | 25 | 25 | 25 | 25 | 25 | 25 | 25 | 25 |
| Benzylalkohol | 0.9 | 0.9 | 0.9 | 0.9 | 0.9 | 0.9 | 0.9 | 0.9 | 0.9 |
| Aluminiumtristearat | 0.4 | 0.4 | 0.4 | 0.4 | 0.4 | 0.4 | 0.4 | 0.4 | 0.4 |
| Span 85 | 0.5 | 0.5 | 0.5 | 0.5 | 0.5 | 0.5 | 0.5 | 0.5 | 0.5 |
| Arginin | - | - | - | - | - | - | 1 | 3 | 2 |
| Lysin | - | - | - | 1 | 3 | 2 | - | - | - |
| Trometamol | 1 | 3 | 2 | - | - | - | - | - | - |
| Propylenglycoldicaprilat | 100ml | 100ml | 100ml | 100ml | 100ml | 100ml | 100ml | 100ml | 100ml |

Tabelle IV

| Verschiedene Konzentrationen des Puffers | | | | | | |
|---|---|---|---|---|---|---|
| Zusammensetzung | Beispiele | | | | | |
| | 22 | 23 | 24 | 25 | 26 | 27 |
| Tylosin Base (5 to 20 $\mu$m) | 25 | 25 | 25 | 25 | 25 | 25 |
| Benzylalkohol | 0.9 | 0.9 | 0.9 | 0.9 | 0.9 | 0.9 |
| Aluminiumtristearat | 0.4 | 0.4 | 0.4 | 0.4 | 0.4 | 0.4 |
| Span 85 | 0.5 | 0.5 | 0.5 | 0.5 | 0.5 | 0.5 |
| Trometamol | 1 | 1.2 | 1.4 | 1.5 | 1.6 | 1.8 |
| Propylenglycoldicaprilat | 100ml | 100ml | 100ml | 100ml | 100ml | 100ml |

Tabelle V

| Verschiedene Lösungsmittel | | | | | | | |
|---|---|---|---|---|---|---|---|
| Zusammensetzung | Beispiele | | | | | | |
|  | 28 | 29 | 30 | 31 | 32 | 33 | 34 |
| Tylosin Base (5 to 20 μm) | 25 | 25 | 25 | 25 | 25 | 25 | 25 |
| Benzylalkohol | 0.9 | 0.9 | 0.9 | 0.9 | 0.9 | 0.9 | 0.9 |
| Aluminiumtristearat | 0.4 | 0.4 | 0.4 | 0.4 | 0.4 | 0.4 | 0.4 |
| Span 85 | 0.5 | 0.5 | 0.5 | 0.5 | 0.5 | 0.5 | 0.5 |
| Trometamol | 1.2 | 1.2 | 1.2 | 1.2 | 1.2 | 1.2 | 1.2 |
| Caprylsäuretriglyceride/Propylenglycoldicaprilate (Summe = 100 ml) | 90/10 | 80/20 | 60/40 | 40/60 | 30/70 | 20/80 | 10/90 |

Die physikalischen Eigenschaften der in den Beispielen 20 und 21 angegebenen Zubereitungen (Tabelle III) waren unzulänglich, so daß die Proben für die Stabilitätstest (siehe Tabelle VI bis IX) nicht verwendet werden konnten.

Nach 24 h wurde die Stabilität der verschiedenen Zubereitungen (Tabelle I bis V, Beispiele 1 bis 34) getestet. Die Ergebnisse sind den Tabellen VI bis X zu entnehmen.

Es zeigte sich, daß mit einer Partikelgröße von 5 bis 20 μm, wobei größer 80 % der Teilchen in diesem Größenbereich liegen, eine homogene Lösung herstellbar ist (Tabelle I, Beispiel 3). Die Lösung zeigt noch eine geringe Neigung zum Verkleben der Partikel. Die Redispergierbarkeit dieser Zubereitung ist noch unbefriedigend (Tabelle VI), so daß diese Zubereitung insgesamt noch als Beurteilung ein "schlecht ++" erhielt. Trotzdem wurde diese Partikelgröße für die weiteren Zubereitungen verwendet.

Tabelle VII zeigt die Ergebnisse mit den unterschiedlichen grenzflächenaktiven Substanzen sowie deren unterschiedliche Mengen. Insgesamt waren die Ergebnisse mit diesen Zubereitungen deutlich besser als mit den Zubereitungen aus Tabelle I. Die besten Ergebnisse zeigte die Zubereitung von Beispiel Nr. 5. Durch Zugabe von grenzflächenaktiven Substanzen konnte ein Verkleben des Wirkstoffes verhindert werden.

Es zeigte sich, daß sich durch Zugabe eines Puffers die Zubereitungen homogen wurden, deutlich besser redispergieren waren und nur noch geringe Neigungen zur Sedimentation zeigten (Tabelle VIII). Die Injizierbarkeit war jedoch noch unbefriedigend.

Die in Beispiel 23 (Tabelle IV) angegebene Zubereitung eine gute Stabilität nach 24 Stunden. Die Lösung war homogen, zeigte keine Sedimetation, ließ sich ausgezeichnet redispergieren und gut injizieren.

Durch Variation des Lösungsmittelsystems (Tabelle X) konnte keine Verbesserung mehr gegenüber Beispiel 23 erzielt werden. Alle in Tabelle X gezeigten Zubereitungen zeigten gute Gesamtergebnisse. Auch wenn die Redispergierbarkeit nicht optimal war.

Mit den in den Beispielen 5, 23 und 28 gezeigten Zubereitungen wurden folgende weitere Stabilitätstests durchgeführt:

a) Raumtemperatur (20-25°C) über 2 Monate
b) Temperatur über 35°C während 1 und 2 Monate
c) Temperatur von 5°C während 1 Monat.

In den Tabellen XI bis XIV wird die Stabilität der Zubereitungen nach Lagerung bei den oben genannten unterschiedlichen Temperaturen und unterschiedlichen Zeiten wiedergegeben.

Die in Beispiel 23 wiedergegebene Zubereitung wies die besten Eigenschaften auf.

Tabelle VI

| Stabilitätstest nach 24 Stunden, Partikelgröße | | | | | | |
|---|---|---|---|---|---|---|
| N. | Galenische Eigenschaften der Zubereitungen | | | Viskosität (cps) | Injizierbarkeit (15/10,18/10) | Gesamtergebnis |
| Beispiele | Homogenität | Sedimentation | Redispergierbarkeit | | | |
| 1 | gleichmäßig | rasches verkleben | schwierig ++ | 500 to 1000 | verstopfen der Nadel | schlecht +++ |
| 2 | gleichmäßig | rasches verkleben | schwierig ++ | 500 to 1000 | verstopfen der Nadel | schlecht +++ |
| 3 | gut | langsames verkleben | Schwierig + | 500 to 1000 | gut | schlecht ++ |

Table VII

| Stabilitätstest nach 24 Stunden, unterschiedliche grenzflächenaktive Substanzen | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergebnis |
| | Homogenität | Sedimentation | Redispergierbarkeit | | | |
| 4 | gleichmäßig | schnell | schwierig ++ | >100 | schwierig ++ | schlecht + |
| 5 | gut | langsam | leicht | >100 | schwierig + | gut + |
| 6 | gut | langsam | schwierig + | >100 | schwierig + | schlecht +++ |
| 7 | gleichmäßig | schnell | schwierig + | >100 | " | " |
| 8 | " | langsam | leicht | >100 | " | gut + |
| 9 | " | langsam | schwierig + | >100 | " | schlecht + |
| 10 | " | schnell | schwierig ++ | >100 | " | schlecht ++ |
| 11 | " | " | " | >100 | " | " |
| 12 | " | " | " | >100 | " | " |

Table VIII

| Stabilitätstest nach 24 Stunden, verschiedene Puffer | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergebnis |
| | Homogenität | Sedimentation | Redispergierbarkeit | | | |
| 13 | gut | langsam | gut | 800 to 1200 | gut | gut (noch zu viskos) |

Table VIII (fortgesetzt)

| Stabilitätstest nach 24 Stunden, verschiedene Puffer | | | | | |
|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 14 | " | " | schwierig | >1500 | schwierig + | schlecht + |
| 15 | " | " | " | 500 to 1000 | schwierig + | schlecht + |
| 16 | " | " | " | 500 to 1000 | " | schlecht + |
| 17 | " | " | " | >1500 | schwierig ++ | schlecht ++ |
| 18 | " | " | " | >1500 | schwierig ++ | " |
| 19 | " | " | " | >1500 | schwierig + | " |

Tabelle IX

| Stabilitätstest nach 24 Stunden, Pufferkonzentration | | | | | |
|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 22 | gut | geringe | gut | 800 to 1000 | gut | gut + |
| 23 | " | keine | ausgezeichnet | 500 to 800 | " | gut ++ |
| 24 | " | " | gut | 500 to 1000 | " | gut + |
| 25 | " | " | " | " | " | gut |
| 26 | " | " | " | " | schwierig | gut - |
| 27 | " | " | " | " | schwierig | " |

Tabelle X

| Stabilitätstest nach 24 Stunden, Lösungsmittel | | | | | |
|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 28 | gut | keine | schwierig | 400 to 800 | gut | gut |
| 29 | gut | " | " | " | " | " |
| 30 | " | " | " | 600 to 1000 | " | " |
| 31 | " | " | " | " | " | " |
| 32 | " | " | " | " | " | " |
| 33 | " | gering | " | " | schwierig | " |

Tabelle X (fortgesetzt)

| Stabilitätstest nach 24 Stunden, Lösungsmittel | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 34 | " | gering | " | " | " | " |

Tabelle XI

| Stabilitätstest - Raumtemperatur 20 bis 25°C über 2 Monate | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 21 | Präzipitation wurde beob-achtet | gleichmäßig | gut | 1000 to 1500 | schwierig | schlecht |
| 41 | homogen | gering | ausgezeichnet | 500 to 800 | gut | gut ++ |
| 50 | " | gleichmäßig | schwierig | 400 to 800 | gut | gut |

Tabelle XII

| Stabilitätstest - 35°C über 1 Monat | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 21 | Homogen | gleichmäßig | gut | 500 to 1000 | gut | gut |
| 41 | Homogen | keine | " | 400 to 600 | " | gut + |
| 50 | " | gleichmäßig | schwierig | 300 to 500 | " | schlecht |

Tabelle XIII

| Stabilitätstest - 35°C über 2 Monate | | | | | | |
|---|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 21 | Homogen | gleichmäßig | gut | 400 to 800 | gut | schlecht |
| 41 | Homogen | keine | " | 400 to 600 | " | gut + |

Tabelle XIII (fortgesetzt)

| Stabilitätstest - 35°C über 2 Monate | | | | | |
|---|---|---|---|---|---|
| N. Beispiele | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 50 | "(Yellow)" | gleichmäßig | schwierig | 200 to 400 | " | schlecht |

Tabelle XIV

| Stabilitätstest - 5°C über 1 Monat | | | | | |
|---|---|---|---|---|---|
| N. Formula | Galenische Eigenschaften der Zubereitungen | | | Viskosität (cps) | Injizierbarkeit (15/10,18/10) | Gesamtergeb-nis |
| | Homogenität | Sedimentation | Redispergier-barkeit | | | |
| 21 | Präzipitation wurde beob-achtet | gleichmäßig | gut | 1000 to 1500 | schwierig | schlecht |
| 41 | Homogen | gleichmäßig | ausgezeichnet | 500 to 1000 | gut | gut ++ |
| 50 | " | " | schwierig | 500 to 1000 | gut | schlecht |

**Patentansprüche**

1. Pharmazeutische Zubereitung mit verzögerter Wirkstofffreigabe enthaltend

   a) einen Wirkstoff
   b) ein gelbildendes Mittel
   c) einen Puffer
   d) eine grenzflächenaktive Substanz.

2. Pharmazeutische Zubereitung nach Anspruch 1, dadurch gekennzeichnet, daß als Wirkstoff Tylosin, Tetracyclin, Oxytetracyclin, Gentamycin, 2-4-Dimethylbenylpyrimidin, deren Salze oder deren Mischungen verwendet werden.

3. Pharmazeutische Zubereitung nach Anspruch 1, dadurch gekennzeichnet, daß als gelbildendes Mittel ein Aluminiumsalz einer Fettsäure enthalten ist.

4. Pharmazeutische Zubereitung nach Anspruch 3, dadurch gekennzeichnet, daß ein Puffer in einer Menge verwendet wird, daß der pH in einem Bereich von pH 4 bis 10 gehalten wird.

5. Pharmazeutische Zubereitung nach Anspruch 1 oder 3, dadurch gekennzeichnet, daß als Puffer ein Tris-Puffer verwendet wird.

6. Pharmazeutische Zubereitung nach Anspruch 1, dadurch gekennzeichnet, daß als grenzflächenaktive Substanz nichtionische, amphotere, anionische oder kationische Emulgatoren verwendet werden.

7. Pharmazeutische Zubereitung nach Anspruch 1, dadurch gekennzeichnet, daß zusätzlich ein physiologisch verträgliches Lösungsmittel oder Lösungsmittelsystem enthalten ist.

8. Pharmazeutische Zubereitung nach Anspruch 7, dadurch gekennzeichnet, daß als Lösungsmittel oder Losungsmittelsystem ein- oder mehrwertige Alkohole, deren Ether, Ester oder deren Mischungen verwendet werden.

9. Pharmazeutische Zubereitung nach Anspruch 7 oder 8, dadurch gekennzeichnet, daß als Lösungsmittel oder Lösungsmittelsystem Benzylalkohol, Propylenglycoldicaprylat, Propylenglycoldicaprat oder deren Mischungen verwendet werden.

10. Pharmazeutische Zubereitung nach Anspruch 1, enthaltend

> 5,0 - 30 Gew.-% Tylosin
> 0,5 - 2 Gew.-% Benzylalkohol
> 0,1 - 3 Gew.-% Aluminium Tristearat
> 0,05 - 5 Gew.-% Span 85
> 0,1 - 3 Gew.-% Tris-Puffer
> 57 - 95 Gew.-% Propylenglycoldicaprylat/-dicaprat,

mit der Maßgabe, daß sich die Summe der einzelnen Komponenten zu 100 Gew.-% addiert.

11. Verwendung einer pharmazeutischen Zubereitung gemäß den Ansprüchen 1 bis 10 zur Herstellung einer Injektionslösung zur therapeutischen Behandlung von Tieren.

**Europäisches Patentamt**

## EUROPÄISCHER RECHERCHENBERICHT

Nummer der Anmeldung

### EINSCHLÄGIGE DOKUMENTE

EP 98106425.6

| Kategorie | Kennzeichnung des Dokuments mit Angabe, soweit erforderlich, der maßgeblichen Teile | Betrifft Anspruch | KLASSIFIKATION DER ANMELDUNG (Int Cl 6) |
|---|---|---|---|
| X,D | GB 2136293 A (RICHTER GEDEON VEGYESZETI GYAR RT) 19. September 1984 (19.09.84), Ansprüche 1,5, Beispiele 1-6, Seite 2, Zeilen 54-61. | 1,2,6, 7,11 | A 61 K 9/06 A 61 K 31/71 A 61 K 31/65 A 61 K 31/505 A 61 K 47/00 A 61 K 45/08 |
| Y | Anspruch 1, Beispiele 1-6, Seite 2, Zeilen 54-61. -- | 3,4,8, 9 | |
| Y,D | WO 94/21267 A1 (NORBROOK LABORATORIES LIMITED) 29. September 1994 (29.09.94), Zusammenfassung, Ansprüche 1-14. -- | 3,4,8, 9 | |
| A,D | WO 96/01634 A1 (NORBROOK LABORATORIES LIMITED) 25. Januar 1996 (25.01.96), Zusammenfassung, Ansprüche 1-6,10, Seite 5, Zeilen 14-17. -- | 1,2,7, 8,11 | |
| A | US 5075295 A (ZUPAN, J.A. et al.) 24. Dezember 1991 (24.12.91), Ansprüche 1,2,4,5, Spalte 1, Zeilen 53-64, Spalte 2, Zeilen 9-18, Beispiel 12. -- | 1,2,5, 7,8,11 | |
| A | BE 825656 A (CIBA-GEIGY AG) 18. August 1975 (18.08.75), Ansprüche 1-6. -- | 1,2,5, 7,8,11 | |
| A,D | US 4018889 A (ARMSTRONG, W.W.) 19. April 1977 (19.04.77), Ansprüche 1-4,7,8, Beispiele | 1,2,5, 7,11 | |

**RECHERCHIERTE SACHGEBIETE (Int. Cl 6)**

A 61 K

Der vorliegende Recherchenbericht wurde für alle Patentansprüche erstellt.

| Recherchenort | Abschlußdatum der Recherche | Prüfer |
|---|---|---|
| WIEN | 12-06-1998 | MAZZUCCO |

Europäisches
Patentamt

**EUROPÄISCHER RECHERCHENBERICHT**

Nummer der Anmeldung

| | EINSCHLÄGIGE DOKUMENTE | | | EP 98106425.6 |
|---|---|---|---|---|
| Kategorie | Kennzeichnung des Dokuments mit Angabe, soweit erforderlich, der maßgeblichen Teile | | Betrifft Anspruch | KLASSIFIKATION DER ANMELDUNG (Int. Cl 6) |
| | 10-12. | | | |
| | ---- | | | |
| | | | | **RECHERCHIERTE SACHGEBIETE** (Int. Cl 6) |

Der vorliegende Recherchenbericht wurde für alle Patentansprüche erstellt.

| Recherchenort | Abschlußdatum der Recherche | Prüfer |
|---|---|---|
| WIEN | 12-06-1998 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82